# EUROPÄISCHE PATENTANMELDUNG

(11) **EP 3 593 865 A1**
(43) Veröffentlichungstag der Anmeldung: **15.01.2020**
(21) Anmeldenummer: 18183142.1
(22) Anmeldetag: 12.07.2018
(51) Int. Cl.: A61Q 19/00, A61K 8/81, C08L 33/10

(54) **EIN MITTEL ZUR ERHÖHUNG DER VISKOSITÄT EINES ÖLS**

(71) Anmelder: BASF SE, 67056 Ludwigshafen am Rhein (DE)
(72) Erfinder: STACHOWIAK, Jan Mirko, 41462 Neuss (DE); TWARDAWSKI, Erik, 40589 Düsseldorf- Holthausen (DE); BUSCH, Swantje, 40589 Düsseldorf-Holthausen (DE); WEISSENEGGER, Markus, 40589 Düsseldorf-Holthausen (DE); BETTHAUSEN, Eva Maria, 67056 Ludwigshafen (DE); SCHUNICHT, Christoph, 40589 Düsseldorf-Holthausen (DE)
(74) Vertreter: BASF IP Association

(57) **Zusammenfassung**

Die vorliegende Erfindung betrifft eine Zusammensetzung enthaltend ein Polymer A, welches enthält 15,5 - 16,5 mol-% Wiederholungseinheiten abgeleitet von Methacrylsäure und 83,5 - 84,5 mol-% Wiederholungseinheiten abgeleitet von 2-Ethylhexylacrylat oder von Stearylmethacrylat oder von Mischungen der beiden, und ein Polymer B, welches enthält 17,5 - 18,5 mol-% Wiederholungseinheiten abgeleitet von 2-N,N-Dimethylaminoethylmethacrylat und 83,5 - 84,5 mol-% Wiederholungseinheiten abgeleitet von 2-Ethylhexylacrylat oder von Stearylmethacrylat oder von Mischungen der beiden, und ein Öl. Weiterhin betrifft die vorliegende Erfindung ein Kit of Parts umfassend ein erstes Behältnis enthaltend Polymer A wie oben definiert in einem Öl, und ein zweites Behältnis enthaltend Polymer B wie oben definiert in demselben Öl, wobei die beiden Behältnisse optional auch zu einem Behältnis mit zwei Kammern verbunden sein können. Weiterhin betrifft die vorliegende Erfindung ein Verfahren zur Herstellung eines Polymers A oder eines Polymers B, beide wie oben definiert, umfassend die Polymerisation der Monomere, welche das Polymer aufbauen, in einem Lösungsmittel, wobei dieses Lösungsmittel eine Mischung ist aus einem Öl und aus mindestens 4 Gew.-%, bezogen auf das Lösungsmittel, eines mono- oder difunktionellen Alkohols mit mindestens 4 C-Atomen.

## Beschreibung

Die vorliegende Erfindung betrifft eine Zusammensetzung enthaltend ein Polymer A, welches enthält 15,5 - 16,5 mol-% Wiederholungseinheiten abgeleitet von Methacrylsäure und 83,5 - 84,5 mol-% Wiederholungseinheiten abgeleitet von 2-Ethylhexylacrylat oder von Stearylmethacrylat oder von Mischungen der beiden, und ein Polymer B, welches enthält 17,5 - 18,5 mol-% Wiederholungseinheiten abgeleitet von 2-N,N-Dimethylaminoethylmethacrylat und 83,5 - 84,5 mol-% Wiederholungseinheiten abgeleitet von 2-Ethylhexylacrylat oder von Stearylmethacrylat oder von Mischungen der beiden, und ein Öl. Weiterhin betrifft die vorliegende Erfindung ein Kit of Parts umfassend ein erstes Behältnis enthaltend Polymer A wie oben definiert in einem Öl, und ein zweites Behältnis enthaltend Polymer B wie oben definiert in demselben Öl, wobei die beiden Behältnisse optional auch zu einem Behältnis mit zwei Kammern verbunden sein können. Weiterhin betrifft die vorliegende Erfindung ein Verfahren zur Herstellung eines Polymers A oder eines Polymers B, beide wie oben definiert, umfassend die Polymerisation der Monomere, welche das Polymer aufbauen, in einem Lösungsmittel, wobei dieses Lösungsmittel eine Mischung ist aus einem Öl und aus mindestens 4 Gew.-%, bezogen auf das Lösungsmittel, eines mono- oder difunktionellen Alkohols mit mindestens 4 C-Atomen.

Zusammensetzungen, welche ein Öl enthalten werden vielfach in der Kosmetik eingesetzt, z. B. als Massageöl. Auch in anderen Anwendungsbereichen werden ölhaltige Zusammensetzungen verwendet. Oft ist es wünschenswert, die Viskosität dieser Zusammensetzungen zu erhöhen. Dies kann durch Zusatzstoffe erfolgen, die als Verdicker bezeichnet werden. Bei diesen Verdickern kann es sich um polymere Substanzen handeln.

US 5,519,063 offenbart kosmetische Formulierungen, die ein Öl enthalten. Zur Verdickung des Öls bzw. der Formulierung enthaltend das Öl enthalten diese Formulierungen zusätzlich eine Kombination aus zwei Polymeren. Eines dieser beiden Polymere enthält in einpolymerisierter Form lipophile Monomere (z. B. Stearylmethacrylat (SMA)) und ein saures Monomer (z. B. Methacrylsäure (MAA)). Das andere enthält in einpolymerisierter Form lipophile Monomere (z. B. Stearylmethacrylat (SMA)) und ein basisches Monomer (z. B. 2-N,N-Dimethylaminoethylmethacrylat (DAMEMA)). In der Beschreibung wird ein Bereich von 3:1 bis 1:3 für das Massenverhältnis von saurem Polymer zu basischem Polymer genannt. Der Anteil der sauren Monomere (sM) und der basischen Monomere (bM), jeweils in mol-%, in den beiden Polymeren, die zusammen verwendet werden, ist in den Beispielen zum Teil gleich und zum Teil verschieden. So beschreibt zum Beispiel die Formulierung F6 eine Kombination der Polymere 3 und 16. Bei diesen beiden Polymeren ist der Anteil an saurem bzw. basischen Monomer gleich 12,8 bzw. 17,1 mol.-%.

Im vorliegenden Text werden saure Monomere zum Teil auch als anionische Monomere bezeichnet, obwohl diese nur nach Deprotonierung Anionen sind. Ebenso werden basische Monomere zum Teil auch als kationische Monomere bezeichnet, obwohl sie erst nach Protonierung Kationen sind. Für Polymere gilt entsprechendes.

Der vorliegenden Erfindung lag die Aufgabe zugrunde, ein Verdickersystem auf Basis von Polymeren für Öle und ölbasierte Formulierungen bereit zu stellen. Dabei sollte eine Verdickung verschiedener Öle möglich sein, möglichst unabhängig von Struktur und Polarität der Öle. Verdickung ist hier gleichbedeutend mit Viskositätserhöhung. Es soll dabei möglich sein, die Öle oder Formulierungen auf einen für kosmetische Anwendungen relevanten Viskositätsbereich einzustellen. Dies soll bevorzugt möglich sein, ohne dass unerwünschte Trübungen oder Phasenseparationen auftreten. Weiterhin soll dies bevorzugt so möglich sein, dass das Verdickersystem selbst in flüssiger Form bereitgestellt werden kann, so dass es leicht verarbeitet und leicht in die Öle oder Formulierungen eingearbeitet werden kann, wobei weiterhin bevorzugt diese flüssige Form, keine für kosmetische Anwendungen unerwünschten Komponenten enthalten soll.

Diese Aufgabe wurde gelöst durch eine Zusammensetzung enthaltend ein Polymer A, welches enthält 15,5 - 16,5 mol-% Wiederholungseinheiten abgeleitet von Methacrylsäure und 83,5 - 84,5 mol-% Wiederholungseinheiten abgeleitet von 2-Ethylhexylacrylat oder von Stearylmethacrylat oder von Mischungen der beiden, und ein Polymer B, welches enthält 17,5 - 18,5 mol-% Wiederholungseinheiten abgeleitet von 2-N,N-Dimethylaminoethylmethacrylat und 83,5 - 84,5 mol-% Wiederholungseinheiten abgeleitet von 2-Ethylhexylacrylat oder von Stearylmethacrylat oder von Mischungen der beiden, und ein Öl. Diese Zusammensetzung ist ein Gegenstand der vorliegenden Erfindung.

In einer Ausführungsform der vorliegenden Erfindung ist diese Zusammensetzung eine kosmetische Zusammensetzung, die optional weitere, bekannte kosmetische Inhaltsstoffe enthält.

In einer Ausführungsform der vorliegenden Erfindung enthält die erfindungsgemäße Zusammensetzung 0,1 bis 10 Gew.-%, bevorzugt 0,5 bis 5 Gew.-% Polymer A, 0,1 bis 10 Gew.-%, bevorzugt 0,5 bis 5 Gew.-% Polymer B, 5 bis 95 Gew.-%, bevorzugt 10 bis 90 Gew.-% Öl, und 0 bis 94,8 Gew.-%, bevorzugt 0 bis 89 Gew.-% weitere, bekannte kosmetische Inhaltsstoffe.

In einer Ausführungsform der vorliegenden Erfindung enthält die erfindungsgemäße Zusammensetzung die Polymere A und B im Massenverhältnis Polymer A zu Polymer B = 1:3 bis 1:4.

Weiterhin ist ein Gegenstand der vorliegenden Erfindung ein Kit of Parts umfassend ein erstes Behältnis enthaltend Polymer A wie oben definiert in einem Öl, und ein zweites Behältnis enthaltend Polymer B wie oben definiert in demselben Öl, wobei die beiden Behältnisse optional auch zu einem Behältnis mit zwei Kammern verbunden sein können.

Weiterhin ist ein Gegenstand der vorliegenden Erfindung ein Verfahren zur Herstellung eines Polymers A oder eines Polymers B, beide wie oben, umfassend die Polymerisation der Monomere, welche das Polymer aufbauen, in einem Lösungsmittel, wobei dieses Lösungsmittel eine Mischung ist aus einem Öl und aus mindestens 4 Gew.-%, bezogen auf das Lösungsmittel, eines mono- oder difunktionellen Alkohols mit mindestens 4 C-Atomen.

In einer Ausführungsform der vorliegenden Erfindung wird der Alkohol in dem erfindungsgemäßen Verfahren ausgewählt aus der Gruppe bestehend aus Glycerinmonooleat, 2-Octyldodecan-1-ol und 2-Hexyldecan-1-ol.

Ein Öl im Sinne der vorliegenden Erfindung kann jedes Öl sein. Gemäß der vorliegenden Erfindung ist ein Öl eine Substanz, die bei 20 °C flüssig ist, und die nicht hydrophil ist. Hydrophil ist eine Substanz, die bei 20 °C mit Wasser in jedem Verhältnis mischbar ist, wobei die resultierende Mischung einphasig ist.

Beispiele für Öle sind Mineralöle, synthetische Öle, Alkylester von Fettsäuren, wobei der Alkylrest bevorzugt 1 bis 30 C-Atome hat und die Fettsäure bevorzugt mindestens 6 C-Atome hat. Weitere Beispiele für Öle sind nicht hydrophile Alkylacetate, zum Beispiel Cyclohexylacetat.

Ein Öl im Sinne der vorliegenden Erfindung kann ein Stoff sein, der ausgewählt wird aus der Gruppe bestehend aus Undecan im Gemisch mit Tridecan, Dioctylether, Bis-(2-propylheptyl)-carbonat, Hexadecyl-2-ethylhexanoat, Dioctylcarbonat, Dodecyldecanoat im Gemisch mit Dodecylctanoat, 2-Ethylhexylstearat, 11-(2,3-dihydroxypropoxycarbonyl)-heptadecanoat, Hexadecyl-7-methyloctanoat, Propan-2-yl-hexadecanoat, Alkyl-(C12/15)-Benzoat, Paraffin, Octan/decan-säure-triglycerid, 2-Decanoyloxypropyl-decanoat im Gemisch mit 2-octanoyloxypropyl-octanoat, (9Z)-Octadec-9-en-säure, Prunus Amygdalus Dulcis, Simmondsia Chinensis Seed oil, und Mischungen der genannten Substanzen.

Die erfindungsgemäßen Verdickersysteme aus zwei Polymeren können für kosmetische, ölhaltige Formulierungen verwendet werden. Sie können auch für andere, ölhaltige Systeme verwendet werden.

Weitere, bekannte kosmetische Inhaltsstoffe sind dem Fachmann bekannt. Sie können einschlägigen Monografien entnommen werden, z. B. Umbach, Kosmetik, Thieme Verlag, 2. Auflage, 1995 und weiteren Monografien, insbesondere solchen jüngeren Datums.

### Allgemeines

- % bedeutet Gew.-%, wenn nichts anderes angegeben ist.
- Alle Messungen wurden bei 23 °C, durchgeführt, wenn nichts anderes angegeben ist.
- Viskositätsmessungen wurden mit einem Brookfield-Viskosimeter, RV-DV I, Sp. TC93, 4 UpM mit Helipath durchgeführt, wenn nichts anderes angegeben ist.
- Die mittleren Molgewichte der Polymere wurden per GPC (kalibriert mit Polymethylmethacrylat) im Lösungsmittel Tetrahydrofuran bestimmt, wenn nichts anderes angegeben ist.

### Abkürzungen

Die folgenden im Text verwandten Abkürzungen stehen für:
- EHA = Ethylhexylacrylat
- SMA = Stearylmethacrylat
- MAA = Methacrylsäure
- DMAEMA = 2-N,N-Dimethylaminoethylmethacrylat
- GMO = Glycerinmonooleat
- n.b. = nicht bestimmt
- AS = Aktivsubstanz
- Mw = Massenmittel der Molmasse; wenn keine Einheit angegeben ist, dann gilt g/mol

Die ersten Versuche wurden auf Basis eines Verdickersystems bestehend aus zwei Polymeren (siehe Tabelle 1) gestartet. Polymer A1 enthielt 16 mol-% MAA, 21 mol-% EHA, 63 mol-% SMA und war also ein anionisches Polymer. Polymer B1 enthielt 16 mol-% DMAEMA, 42 mol-% EHA, 42 mol-% SMA und war also ein kationisches Polymer. Beide Polymere lagen jeweils in einer Konzentration von 30,0 % in Methylbenzoat als Lösungsmittel vor, in dem die Polymere mittels Lösungspolymerisation synthetisiert wurden.

**Tabelle 1: Zusammensetzung der Polymere A1 und B1**

| **mol-%** | **Polymer A1** | **Polymer B1** |
|---|---|---|
| EHA | 21 | 42 |
| SMA | 63 | 42 |
| MAA | 16 | - |
| DMAEMA | - | 16 |
| | | |

| **Gew-%** | | |
|---|---|---|
| Polymer | 30 | 30 |
| Methylbenzoat | 70 | 70 |
| | | |

| **Mw** | | |
|---|---|---|
| | 200.000 | 300.000 |

Diese Polymerlösungen waren bei Raumtemperatur flüssig und wurden mit 5,0 % AS Gesamtpolymer (2,5 % AS Polymer A1 mit 16 mol-% MAA und 2,5 % AS Polymer B1 mit 16 mol-% DMAEMA, im Gewichtsverhältnis 1:1) unter Rühren in die zu verdickende Matrix (siehe Tabelle 2) eingearbeitet, wobei zuerst Polymer A1 bei Raumtemperatur im zu verdickenden Öl gelöst wurde und anschließend Polymer B1 unter Rühren zugegeben wurde.

**Tabelle 2: 5,0 % AS Gesamtpolymer (2,5 % AS Polymer A1, 16 mol-% MAA und 2,5 % AS Polymer B1, 16 mol-% DMAEMA, Lösungsmittel: Methylbenzoat) in verschiedenen Ölen**

| **Öl (IUPAC oder INCI)** | **Handelsname** | **Viskosität [mPas]** | **Beobachtung** |
|---|---|---|---|
| Undecan (und) Tridecan | Cetiol® Ultimate | 118.000 | Klar |
| Dioctylether | Cetiol® OE | 8.500 | Klar |
| Bis(2-propylheptyl) carbonat | Cetiol® 4 All | 76.000 | Klar |
| Hexadecyl 2-ethylhexanoat | Luvitol® EHO | 73.000 | Klar |
| Dioctylcarbonat | Cetiol® CC | 58.000 | Klar |
| Dodecyl decanoate / Dodecyl octanoat | Cetiol® C5 | 30.000 | Klar |
| 2-Ethylhexylstearat | Cetiol® 868 | 75.000 | Klar |
| 11-(2,3-dihydroxypropoxycarbonyl) heptadecanoate | Cetiol® LC | 13.500 | Klar |
| Hexadecyl 7-methyloctanoate | Cetiol® SN | 75.000 | Klar |
| Propan-2-yl hexadecanoat | IPP | 52.000 | Klar |
| Propan-2-yl tetradecanoat | IPM | 41.000 | Klar |
| Propan-2-yl octadecanoat | IPS | 61.000 | Klar |
| Alkyl (C12/15) Benzoat | Cetiol® AB | 57.000 | Klar |
| Dioctylether (und) Dodecanol | Cetiol® LDO | 250 | Klar |
| 1-Octadecanoxy propan-2-ol | Cetiol® E | n.b. | Phasenseparation |
| 2-Octyldodecan-1-ol | Eutanol® G | 500 | Klar |
| 2-Hexyldecan-1-ol | Eutanol® G16 | 50 | Klar, aber keine messbare Verdickung |
| Paraffin | | 140.000 | Klar |
| Octanoic/decanoic acid triglycerid | Myritol® 312 | n.b. | Phasenseparation |
| Octanoic/decanoic acid triglycerid | Myritol® 331 | n.b. | Phasenseparation |
| 2-Decanoyloxypropyl decanoate;2-octanoyloxypropyl octanoate | Myritol® PGDC | n.b. | Phasenseparation |
| Dibutyl hexanedioat | Cetiol® B | n.b. | Phasenseparation |
| [(Z)-octadec-9-enyl] (Z)-docos-13-enoate | Cetiol® J600 | n.b. | Phasenseparation |
| (9Z)-Octadec-9-en säure | Olivenöl | n.b. | Phasenseparation |
| Prunus Amygdalus Dulcis | Mandelöl | n.b. | Phasenseparation |
| Simmondsia Chinensis Seed oil | Jojobaöl | n.b. | Phasenseparation |

Einsatzkonzentrationen von ≤ 4,0 % AS Gesamtpolymer (Polymer A1 mit 16 mol-% MAA und Polymer B1 mit 16 mol-% DMAEMA im Gewichtsverhältnis 1:1) führten häufig zu Instabilität in Form von Phasenseparation in der zu verdickenden Matrix (siehe Tabelle 3).

**Tabelle 3: ≤ 4,0 % AS Gesamtpolymer (Polymer A1, 16 mol-% MAA und Polymer B1, 16 mol-% DMAEMA im Gewichtsverhältnis 1:1, Lösungsmittel: Methylbenzoat) in verschiedenen Ölen**

| **Öl** | **% Polymer A** | **% Polymer B** | **Viskosität [mPas]** | **Beobachtung** |
|---|---|---|---|---|
| 2-Ethylhexylstearat | 0,5 | 0,5 | 4.500 | Phasenseparation |
| 2-Ethylhexylstearat | 1,0 | 1,0 | 10.500 | Phasenseparation |
| 2-Ethylhexylstearat | 1,5 | 1,5 | 18.000 | Phasenseparation |
| 2-Ethylhexylstearat | 2,0 | 2,0 | 55.000 | Klar |
| Dioctylcarbonat | 0,5 | 0,5 | 2.500 | Phasenseparation |
| Dioctylcarbonat | 1,0 | 1,0 | 2.000 | Phasenseparation |
| Dioctylcarbonat | 1,5 | 1,5 | 4.000 | Phasenseparation |
| Dioctylcarbonat | 2,0 | 2,0 | 10.000 | Klar |
| Alkyl (C12/15) Benzoat | 0,5 | 0,5 | 1.250 | Klar |
| Alkyl (C12/15) Benzoat | 1,0 | 1,0 | 2.000 | Klar |
| Alkyl (C12/15) Benzoat | 1,5 | 1,5 | 2.500 | Klar |
| Alkyl (C12/15) Benzoat | 2,0 | 2,0 | 20.000 | Klar |

Der für den Anwendungsbereich in der Kosmetik attraktive Viskositätsbereich von ca.1000 mPas bis ca. 10.000 mPas konnte mit diesem Basissystem nicht in allen Ölen stabil erreicht werden.

Ebenso konnte das beschriebene System (Polymer A plus Polymer B im Gewichtsverhältnis 1:1) keine handelsüblichen synthetischen oder natürlichen Triglyceride verdicken, da es in diesen Ölen ebenfalls zu einer Phasenseparation kam.

Neben den zur Verdickung eingesetzten Polymeren A und B wurde das zu 70,0 % enthaltene Methylbenzoat zusätzlich mit in die zu verdickende Matrix eingebracht. Methylbenzoat sollte in diesen Konzentrationen in der Kosmetik allerdings aufgrund des starken Eigengeruchs sowie aus toxikologischen Gründen nicht zum Einsatz kommen.
Der nächste Entwicklungsschritt bezog sich auf den Austausch von Methylbenzoat hin zu einem für die Kosmetik geeigneten Öl als Lösungsmittel. Der Austausch des Lösungsmittels bei gleichbleibender Konzentration von 30,0 % gelöstem Polymer beeinträchtigte die Synthese von Polymer B nicht.

Anders verhielt es sich bei der Synthese von Polymer A. Versuche, die Polymersynthese von Polymer A in gleicher Konzentration von 30,0 % in verschiedenen Lösungsmitteln durchzuführen, resultierten zum Teil in festen, und deshalb schwierig zu verarbeitenden Produkten, die für die Anwendung als Verdickersystem für kosmetische Produkte nicht, oder jedenfalls nur wenig, geeignet waren (siehe Tabelle 4). Auch eine Verringerung des Aktivsubstanzgehaltes des Polymers auf 20,0 % bei der Polymerisation führte nicht zu einer flüssigen Polymerlösung.

**Tabellen 4 und 4a: Einfluss des Lösungsmittels auf die Viskosität des polymeren Endprodukts nach Synthese**

| **Lösungsmittel für die Polymerisation** | **Handelsname** | **Polymer A (30 %, 16 mol-% MAA)** | **Polymer B (30 %, 16 mol-% DMAEMA)** |
|---|---|---|---|
| Methylbenzoat | | Hochviskos | Leicht viskos |
| Bis(2-propylheptyl) carbonat | Cetiol® 4 All | Fest | Viskos |
| 2-Ethylhexylstearat | Cetiol® 868 | Hochviskos - Fest | Leicht viskos |
| 2-Octyldodecan-1-ol | Eutanol® G | Leicht viskos | Leicht viskos |
| 2-Hexyldecan-1-ol | Eutanol® G16 | Leicht viskos | Leicht viskos |
| Propan-2-yl tetradecanoat | IPM | Fest | Viskos |
| Alkyl (C12/15) Benzoat | Cetiol® AB | Fest | Viskos |
| Octanoic/decanoic acid triglycerid | Myritol® 331 | Hochviskos - Fest | Leicht viskos |
| | | | |

| **Lösungsmittel für die Polymerisation** | **Handelsname** | **Polymer A (20%, 16 mol-% MAA)** | |
|---|---|---|---|
| Bis(2-propylheptyl) carbonat | Cetiol® 4 All | Fest | |
| 2-Ethylhexylstearat | Cetiol® 868 | Hochviskos - Fest | |
| 2-Octyldodecan-1-ol | Eutanol® G | Leicht viskos | |
| 2-Hexyldecan-1-ol | Eutanol® G16 | Leicht viskos | |
| Propan-2-yl tetradecanoat | IPM | Hochviskos - Fest | |
| Alkyl (C12/15) Benzoat | Cetiol® AB | Fest | |
| Octanoic/decanoic acid triglycerid | Myritol® 331 | Leicht viskos | |

Überraschenderweise wurde jedoch gefunden, dass Guerbetalkohole, z.B. 2-Hexyldecan-1-ol und 2-Octyldodecan-1-ol, für die Synthese von Polymer A geeignete Lösungsmittel darstellen. Diese führten bei der Synthese des Polymers als 30,0 %-Lösung in den genannten Guerbetalkoholen nicht zu festen, sondern zu vergleichsweise niedrigviskosen, flüssigen Produkten (10.000 bis 20.000 mPas). Das Polymer im Lösungsmittel erlaubte so eine einfache Einarbeitung in die zu verdickenden kosmetischen Öle oder Formulierungen und blieb gleichzeitig pumpfähig.

Als Ursache für dieses überraschende Ergebnis wird eine Erniedrigung der resultierenden Viskosität durch den Einfluss der Hydroxylgruppen, die als funktionelle Einheit in den Guerbetalkoholen enthalten sind, vermutet.

**Tabelle 5: Zusammensetzung der Polymere A2, A3 und B2, B3 im Lösungsmittel 2-Octyldodecan-1-ol**

| **mol-%** | **Polymer A2** | **Polymer A3** | **Polymer B2** | **Polymer B3** |
|---|---|---|---|---|
| EHA | 21 | 20,5 | 42 | 41 |
| SMA | 63 | 61,5 | 42 | 41 |
| MAA | 16 | 18 | - | - |
| DMAEMA | - | - | 16 | 18 |
| | | | | |

| **Gew-%** | | | | |
|---|---|---|---|---|
| Polymer | 30 | 30 | 30 | 30 |
| 2-Octyldodecan-1-ol | 70 | 70 | 70 | 70 |
| | | | | |

| **Mw** | | | | |
|---|---|---|---|---|
| | 250.000 | 250.000 | 270.000 | 280.000 |

Allerdings zeigten diese Polymere, wie in Tabelle 6 gezeigt, eine signifikant niedrigere Verdickungsleistung als die vergleichbaren Polymere in dem Lösungsmittel Methylbenzoat.

**Tabelle 6: 5,0 % AS Gesamtpolymer (2,5 % AS Polymer A2, 16 mol-% MAA und 2,5 % AS Polymer B2, 16 mol-% DMAEMA, Lösungsmittel: Eutanol® G) in verschiedenen Ölen.**

| **Öl (IUPAC oder INCI)** | **Handelsname** | **Viskosität [mPas]** | **Beobachtung** |
|---|---|---|---|
| Undecan (und) Tridecan | Cetiol® Ultimate | 4.000 | Klar |
| Dioctylether | Cetiol® OE | 1.000 | Klar |
| Bis(2-propylheptyl) carbonat | Cetiol® 4 All | 5.000 | Klar |
| Hexadecyl 2-ethylhexanoat | Luvitol® EHO | 9.000 | Klar |
| Dioctylcarbonat | Cetiol® CC | 1.000 | Klar |
| Dodecyl decanoate / Dodecyl octanoat | Cetiol® C5 | 1.000 | Klar |
| 2-Ethylhexylstearat | Cetiol® 868 | 4.300 | Klar |
| 11-(2,3-dihydroxypropoxycarbonyl) heptadecanoate | Cetiol® LC | 2.000 | Klar |
| Hexadecyl 7-methyloctanoate | Cetiol® SN | 10.000 | Klar |
| Propan-2-yl hexadecanoat | IPP | 1.500 | Klar |
| Propan-2-yl tetradecanoat | IPM | 1.250 | Klar |
| Propan-2-yl octadecanoat | IPS | 2.000 | Klar |
| Alkyl (C12/15) Benzoat | Cetiol® AB | 1.500 | Klar |
| 1-Octadecanoxy propan-2-ol | Cetiol® E | n.b. | Phasenseparation |
| Octanoic/decanoic acid triglycerid | Myritol® 312 | n.b. | Phasenseparation |
| Octanoic/decanoic acid triglycerid | Myritol® 331 | n.b. | Phasenseparation |
| 2-Decanoyloxypropyl decanoate;2-octanoyloxypropyl octanoate | Myritol® PGDC | 1.000 | Klar |
| Dibutyl hexanedioat | Cetiol® B | n.b. | Phasenseparation |
| (9Z)-Octadec-9-en säure | Olivenöl | n.b. | Phasenseparation |
| Prunus Amygdalus Dulcis | Mandelöl | n.b. | Phasenseparation |
| Simmondsia Chinensis Seed oil | Jojobaöl | n.b. | Phasenseparation |

Eine Erhöhung des funktionalen Gehalts auf 18 mol-% MAA in Polymer A3 sowie auf 18 mol-% DMAEMA in Polymer B3 führte dann wieder zu einer Verbesserung der Verdickungsleistung.

**Tabelle 7: 5,0 % AS Gesamtpolymer (2,5 % AS Polymer A3, 18 mol-% MAA und 2,5 % AS Polymer B3, 18 mol-% DMAEMA, Lösungsmittel: Eutanol® G) in verschiedenen Ölen.**

| **Öl (IUPAC oder INCI)** | **Handelsname** | **Viskosität [mPas]** | **Beobachtung** |
|---|---|---|---|
| Undecan (und) Tridecan | Cetiol® Ultimate | 79.000 | Klar |
| Dioctylether | Cetiol® OE | 17.000 | Klar |
| Bis(2-propylheptyl) carbonat | Cetiol® 4 All | 72.000 | Klar |
| Hexadecyl 2-ethylhexanoat | Luvitol® EHO | 72.000 | Klar |
| Dioctylcarbonat | Cetiol® CC | 15.000 | Klar |
| Dodecyl decanoate / Dodecyl octanoat | Cetiol® C5 | 1.250 | Klar |
| 2-Ethylhexylstearat | Cetiol® 868 | 49.000 | Klar |
| 11-(2,3-dihydroxypropoxycarbonyl) heptadecanoate | Cetiol® LC | 27.000 | Klar |
| Hexadecyl 7-methyloctanoate | Cetiol® SN | 76.000 | Klar |
| Propan-2-yl hexadecanoat | IPP | 23.000 | Klar |
| Propan-2-yl tetradecanoat | IPM | 15.200 | Klar |
| Propan-2-yl octadecanoat | IPS | 25.000 | Klar |
| Alkyl (C12/15) Benzoat | Cetiol® AB | 23.000 | Klar |
| 1-Octadecanoxy propan-2-ol | Cetiol® E | n.b. | Phasenseparation |
| Octanoic/decanoic acid triglycerid | Myritol® 312 | n.b. | Phasenseparation |
| Octanoic/decanoic acid triglycerid | Myritol® 331 | n.b. | Phasenseparation |
| 2-Decanoyloxypropyl decanoate;2-octanoyloxypropyl octanoate | Myritol® PGDC | n.b. | Phasenseparation |
| Dibutyl hexanedioat | Cetiol® B | n.b. | Phasenseparation |
| (9Z)-Octadec-9-en säure | Olivenöl | n.b. | Phasenseparation |
| Prunus Amygdalus Dulcis | Mandelöl | n.b. | Phasenseparation |
| Simmondsia Chinensis Seed oil | Jojobaöl | n.b. | Phasenseparation |

Weiterhin wurde der Einfluss weiterer Verbindungen, die ebenfalls eine Hydroxylgruppe tragen, auf die Verdickungsleistung untersucht (siehe Tabelle 8).

**Tabelle 8: 5,0 % AS Gesamtpolymer (2,5 % AS Polymer A1, 16 mol-% MAA und 2,5 % AS Polymer B1, 16 mol-% DMAEMA, Lösungsmittel: Methylbenzoat) in 2-Ethylhexylstearat plus verschiedene Additive**

| **Öl (IUPAC oder INCI)** | **Handelsname** | **Viskosität [mPas]** | **Beobachtung** |
|---|---|---|---|
| 3,3% Glycerin | | 42.500 | Trüb |
| 5,0% Ethanol | | 500 | Klar |
| 6,5% Propan-2-ol | | 500 | Klar |
| 2,0% 1-Hexanol | Lorol® C6 | 62.000 | Klar |
| 14,1% Glycerylmonolaurat | Monomuls®90 L12 | n.b. | Phasenseparation |
| 17,2% 1-Decanol | Lorol® C10 | 500 | Klar |
| 5,0% Glycerylmonooleat | Monomuls® 90 O 18 | 45.000 | Klar |
| 4,0% 1-Dodecanol | Lorol® C12 | 41.000 | Klar |
| 32,4% 2-Octyldodecan-1-ol | Eutanol® G | 10.000 | Klar |
| 26,3% 2-Hexyldecan-1-ol | Eutanol® G16 | 10.000 | Klar |

Somit konnte der Einfluss der Hydroxylgruppen-tragenden Verbindungen auf die Erniedrigung der Viskosität von Polymer A im Lösungsmittel gezeigt werden.

Auf Basis dieser Ergebnisse war es nun möglich, Polymer A in weiteren Lösungsmitteln, neben den reinen, bereits beschriebenen Guerbetalkoholen, zu synthetisieren und flüssige, prozessfähige Produkte zu erhalten, indem eine der in Tabelle 6 aufgeführten Komponenten zugegeben wurde. Besonders bevorzugt erschienen GMO sowie 2-Octyldodecan-1-ol, da diese bereits seit langem in kosmetischen Applikationen angewandt werden.

Der direkte Vergleich zeigte den gefundenen Vorteil: 30,0 % Polymer A in 70,0 % 2-Ethylhexylstearat synthetisiert war nicht prozessfähig. 30,0 % Polymer A in 65,0 % 2-Ethylhexylstearat und 5,0 % GMO synthetisiert war flüssig und niedrigviskos (ca. 10.000 mPas).

Die Verdickungsleistung der in 2-Ethylhexylstearat mit GMO- bzw. 2-Octyldodecan-1-ol-Zusatz synthetisierten Polymervarianten war im Vergleich zu den Polymervarianten in Methylbenzoat allerdings signifikant herabgesetzt wie an den folgenden Beispielen in Tabelle 9 bzw. 10 zu erkennen ist.

**Tabelle 9: Zusammensetzung der Polymere A und B; unterschiedlich in Bezug auf Lösungsmittel bzw. Additiv**

| **mol-%** | **Polymer A1** | **Polymer A4** | **Polymer A5** | **Polymer B1** | **Polymer B4** |
|---|---|---|---|---|---|
| EHA | 21 | 21 | 21 | 42 | 42 |
| SMA | 63 | 63 | 63 | 42 | 42 |
| MAA | 16 | 16 | 16 | - | - |
| DMAEMA | - | - | - | 16 | 16 |
| | | | | | |

| **Gew-%** | | | | | |
|---|---|---|---|---|---|
| Polymer | 30 | 30 | 30 | 30 | 30 |
| Methylbenzoat | 70 | - | - | 70 | - |
| 2-Ethylhexylstearate | - | 65 | 42 | - | 70 |
| GMO | - | 5 | - | - | - |
| 2-Octyldodecan-1-ol | - | - | 28 | - | - |
| | | | | | |

| **Mw** | | | | | |
|---|---|---|---|---|---|
| | 200.000 | 230.000 | 220.000 | 300.000 | 280.000 |

Tabelle 10 zeigt die resultierenden Viskositäten aus Kombinationen der jeweiligen Polymere A und B in 2-Ethylhexylstearate als zu verdickendes Öl, wobei immer 5,0 % AS Gesamtpolymer (2,5 % AS Polymer A mit 16 mol-% MAA und 2,5 % AS Polymer B mit 16 mol-% im Gewichtsverhältnis 1:1) eingesetzt wurden.

**Tabelle 10: Resultierende Viskositäten der Polymerkombinationen aus Tabelle 9 in mPas**

| **Polymer** | **A1** | **A4** | **A5** |
|---|---|---|---|
| **B1** | 75.000 | | |
| **B4** | | 20.000 | 45.000 |

Um die Verdickungsleitung zu optimieren folgten Versuche, Polymer A und Polymer B mit unterschiedlich hohen funktionellen Anteilen zu synthetisieren (siehe Tabelle 9). Im Folgenden wurden Polymer A mit 16 mol-% und 18 mol-% MAA und Polymer B mit 16 mol-% und 18 mol-% DMAEMA synthetisiert und diese in unterschiedlichen Kombinationen als Verdicker getestet, wobei 5,0 % AS Gesamtpolymer eingesetzt wurden und das Gewichtsverhältnis von Polymer A zu Polymer B jeweils 1:1 war.

**Tabelle 11: Zusammensetzung der Polymere A und B mit unterschiedlichen funktionellen Anteilen sowie Lösungsmittel und ggf. Additiv**

| | **Polymer A4** | **Polymer A6** | **Polymer B4** | **Polymer B5** |
|---|---|---|---|---|
| **mol-%** | | | | |
| EHA | 21 | 20,5 | 42 | 41 |
| SMA | 63 | 61,5 | 42 | 41 |
| MAA | 16 | 18 | - | - |
| DMAEMA | - | - | 16 | 18 |
| | | | | |

| **Gew-%** | | | | |
|---|---|---|---|---|
| Polymer | 30 | 30 | 30 | 30 |
| 2-Ethylhexylstearate | 65 | 65 | 70 | 70 |
| GMO | 5 | 5 | - | - |
| | | | | |

| **Mw** | | | | |
|---|---|---|---|---|
| | 230.000 | 220.000 | 280.000 | 270.000 |

**Tabelle 12: 5,0 % AS Gesamtpolymer (2,5 % AS Polymer A4, 16 mol-% MAA und 2,5 % AS Polymer B4, 16 mol-% DMAEMA) in verschiedenen Ölen.**

| **Öl (IUPAC oder INCI)** | **Handelsname** | **Viskosität [mPas]** | **Beobachtung** |
|---|---|---|---|
| Undecan (und) Tridecan | Cetiol® Ultimate | 57.000 | Klar |
| Dioctylether | Cetiol® OE | 45.000 | Klar |
| Bis(2-propylheptyl) carbonat | Cetiol® 4 All | 37.000 | Opak |
| Hexadecyl 2-ethylhexanoat | Luvitol® EHO | n.b. | Phasenseparation |
| Dioctylcarbonat | Cetiol® CC | 25.000 | Opak |
| Dodecyl decanoate / Dodecyl octanoat | Cetiol® C5 | 37.000 | Klar |
| 2-Ethylhexylstearat | Cetiol® 868 | 20.000 | Trüb |
| 11-(2,3-dihydroxypropoxycarbonyl) heptadecanoate | Cetiol® LC | 28.000 | Opak |
| Hexadecyl 7-methyloctanoate | Cetiol® SN | 27.000 | Trüb |
| Propan-2-yl hexadecanoat | IPP | 30.000 | Klar |
| Propan-2-yl tetradecanoat | IPM | 31.000 | Klar |
| Propan-2-yl octadecanoat | IPS | 32.000 | Klar |
| Alkyl (C12/15) Benzoat | Cetiol® AB | 60.000 | Klar |
| 1-Octadecanoxy propan-2-ol | Cetiol® E | n.b. | Phasenseparation |
| Octanoic/decanoic acid triglycerid | Myritol® 312 | 28.000 | Trüb |
| Octanoic/decanoic acid triglycerid | Myritol® 331 | 3.000 | Trüb |
| 2-Decanoyloxypropyl decanoate;2-octanoyloxypropyl octanoate | Myritol® PGDC | n.b. | Phasenseparation |
| Dibutyl hexanedioat | Cetiol® B | n.b. | Phasenseparation |
| (9Z)-Octadec-9-en säure | Olivenöl | n.b. | Phasenseparation |
| Prunus Amygdalus Dulcis | Mandelöl | n.b. | Phasenseparation |
| Simmondsia Chinensis Seed oil | Jojobaöl | n.b. | Phasenseparation |

**Tabelle 13: 5,0 % AS Gesamtpolymer (2,5 % AS Polymer A4, 16 mol-% MAA und 2,5 % AS Polymer B5, 18 mol-% DMAEMA) in verschiedenen Ölen**

| **Öl (IUPAC oder INCI)** | **Handelsname** | **Viskosität [mPas]** | **Beobachtung** |
|---|---|---|---|
| 2-Ethylhexylstearat | Cetiol® 868 | n.b. | Phasenseparation |
| Dioctylether | Cetiol® OE | 41.000 | Trüb |
| 2-Decanoyloxypropyl decanoate;2-octanoyloxypropyl octanoate | Myritol® PGDC | n.b. | Phasenseparation |

**Tabelle 14: 5,0 % AS Gesamtpolymer (2,5 % AS Polymer A6, 18 mol-% MAA und 2,5 % AS Polymer B4, 16 mol-% DMAEMA) in verschiedenen Ölen**

| **Öl (IUPAC oder INCI)** | **Handelsname** | **Viskosität [mPas]** | **Beobachtung** |
|---|---|---|---|
| 2-Ethylhexylstearat | Cetiol® 868 | 59.750 | Trüb |
| 2-Decanoyloxypropyl decanoate;2-octanoyloxypropyl octanoate | Myritol® PGDC | 21.750 | Trüb |

**Tabelle 15: 5,0 % AS Gesamtpolymer (2,5 % AS Polymer A6, 18 mol-% MAA und 2,5 % AS Polymer B5, 18 mol-% DMAEMA) in verschiedenen Ölen**

| **Öl (IUPAC oder INCI)** | **Handelsname** | **Viskosität [mPas]** | **Beobachtung** |
|---|---|---|---|
| Bis(2-propylheptyl) carbonat | Cetiol® 4 All | 93.500 | Trüb |
| 2-Ethylhexylstearat | Cetiol® 868 | 57.000 | Trüb |
| 11-(2,3-dihydroxypropoxycarbonyl) heptadecanoate | Cetiol® LC | 66.000 | Trüb |
| 2-Decanoyloxypropyl decanoate;2-octanoyloxypropyl octanoate | Myritol® PGDC | n.b. | Phasenseparation |

Da es in einer Vielzahl von Kombinationen zu einer Trübung bzw. Phasenseparation kam, wurde versucht die Stabilität der Formulierung zu verbessern, indem ausgehend vom Gewichtsverhältnis von 1:1 der Anteil von Polymer B in der Mischung aus Polymer A und Polymer B sukzessive erhöht wurde.

Überraschenderweise wurde gefunden, dass durch die Variation des Gewichtsverhältnisses von Polymer A mit 16 mol-% MAA zu Polymer B mit 18 mol-% DMAEMA auf einen Wert von 1:3 und 1:4 erreicht wurde, dass verschiedene Öle unterschiedlicher Polarität verdickt werden konnten und dabei eine klare, nicht trübe Mischung aus Öl plus Polymer A und Polymer B erhalten wurde.

**Tabelle 16: 5,0 % AS Gesamtpolymer (1,25 % AS Polymer A4, 16 mol-% MAA und 3,75 % AS Polymer B5, 18 mol-% DMAEMA) in verschiedenen Ölen**

| **Öl (IUPAC oder INCI)** | **Handelsname** | **Viskosität [mPas]** | **Beobachtung** |
|---|---|---|---|
| Undecan (und) Tridecan | Cetiol® Ultimate | 49.000 | Klar |
| Dioctylether | Cetiol® OE | 35.000 | Klar |
| Bis(2-propylheptyl) carbonat | Cetiol® 4 All | 75.000 | Klar |
| Hexadecyl 2-ethylhexanoat | Luvitol® EHO | 38.000 | Klar |
| Dioctylcarbonat | Cetiol® CC | 32.000 | Klar |
| Dodecyl decanoate / Dodecyl octanoat | Cetiol® C5 | 28.000 | Klar |
| 2-Ethylhexylstearat | Cetiol® 868 | 32.000 | Klar |
| 11-(2,3-dihydroxypropoxycarbonyl) heptadecanoate | Cetiol® LC | 31.000 | Klar |
| Hexadecyl 7-methyloctanoate | Cetiol® SN | 48.000 | Klar |
| Propan-2-yl hexadecanoat | IPP | 32.000 | Klar |
| Propan-2-yl tetradecanoat | IPM | 30.000 | Klar |
| Propan-2-yl octadecanoat | IPS | 34.000 | Klar |
| Alkyl (C12/15) Benzoat | Cetiol® AB | 60.000 | Klar |
| 1-Octadecanoxy propan-2-ol | Cetiol® E | n.b. | Phasenseparation |
| Octanoic/decanoic acid triglycerid | Myritol® 312 | 28.000 | Trüb |
| Octanoic/decanoic acid triglycerid | Myritol® 331 | 3.000 | Trüb |
| 2-Decanoyloxypropyl decanoate;2-octanoyloxypropyl octanoate | Myritol® PGDC | 9.000 | Klar |
| Dibutyl hexanedioat | Cetiol® B | n.b. | Phasenseparation |
| (9Z)-Octadec-9-en säure | Olivenöl | n.b. | Phasenseparation |
| Prunus Amygdalus Dulcis | Mandelöl | n.b. | Phasenseparation |
| Simmondsia Chinensis Seed oil | Jojobaöl | n.b. | Phasenseparation |
| Paraffin | | 55.000 | Klar |

**Tabelle 17: 5,0 % AS Gesamtpolymer (1,0 % AS Polymer A4, 16 mol-% MAA und 4,0 % AS Polymer B5, 18 mol-% DMAEMA) in verschiedenen Ölen**

| **Öl (IUPAC oder INCI)** | **Handelsname** | **Viskosität [mPas]** | **Beobachtung** |
|---|---|---|---|
| Octanoic/decanoic acid triglycerid | Myritol® 312 | 2.000 | Opak |
| Octanoic/decanoic acid triglycerid | Myritol® 331 | 1.000 | Opak |
| (9Z)-Octadec-9-en säure | Olivenöl | 2.000 | Klar |
| Prunus Amygdalus Dulcis | Mandelöl | 1.000 | Klar |
| Simmondsia Chinensis Seed oil | Jojobaöl | 5.000 | Klar |

Auch handelsübliche synthetische und natürliche Triglyceride konnten mit dieser Polymerkombination verdickt werden. Überraschenderweise wurde festgestellt, dass auch eine Verdickung ohne Trübung mit ≤ 4,0 % AS ohne die in diesen Ölen vorher aufgetretene Phasenseparation möglich war und somit ein breiter Viskositätsbereich der verdickten Systeme zugänglich ist.

**Tabelle 18: Konzentrationsreihe (Polymer A4, 16 mol-% MAA und Polymer B5, 18 mol-% DMAEMA in unterschiedlichen Gewichtsverhältnissen, Lösungsmittel: Cetiol® 868 +5,0 % GMO) in 2-Ethylhexylstearat**

| **Öl** | **% Polymer A4** | **% Polymer B5** | **Viskosität [mPas]** | **Beobachtung** |
|---|---|---|---|---|
| 2-Ethylhexylstearat | 0,5 | 0,5 | 500 | Klar |
| 2-Ethylhexylstearat | 1,0 | 1,0 | 750 | Klar |
| 2-Ethylhexylstearat | 1,5 | 1,5 | 1.000 | Klar |
| 2-Ethylhexylstearat | 2,0 | 2,0 | 6.000 | Klar |
| 2-Ethylhexylstearat | 2,5 | 2,5 | 32.000 | Klar |
| 2-Ethylhexylstearat | 5,0 | 5,0 | > 250.000 | Klar |

## Patentansprüche

1. Eine Zusammensetzung enthaltend
ein Polymer A, welches enthält
15,5 - 16,5 mol-% Wiederholungseinheiten abgeleitet von Methacrylsäure und
83,5 - 84,5 mol-% Wiederholungseinheiten abgeleitet von 2-Ethylhexylacrylat oder von Stearylmethacrylat oder von Mischungen der beiden,
und ein Polymer B, welches enthält
17,5 - 18,5 mol-% Wiederholungseinheiten abgeleitet von
2-N,N-Dimethylaminoethylmethacrylat und
83,5 - 84,5 mol-% Wiederholungseinheiten abgeleitet von 2-Ethylhexylacrylat oder von Stearylmethacrylat oder von Mischungen der beiden,
und ein Öl.

2. Die Zusammensetzung nach Anspruch 1, wobei diese Zusammensetzung eine kosmetische Zusammensetzung ist und optional weitere, bekannte kosmetische Inhaltsstoffe enthält.

3. Die Zusammensetzung nach einem der Ansprüche 1 oder 2, wobei diese enthält
0,1 bis 10 Gew.-%, bevorzugt 0,5 bis 5 Gew.-% Polymer A,
0,1 bis 10 Gew.-%, bevorzugt 0,5 bis 5 Gew.-% Polymer B,
5 bis 95 Gew.-%, bevorzugt 10 bis 90 Gew.-% Öl, und
0 bis 94,8 Gew.-%, bevorzugt 0 bis 89 Gew.-% weitere, bekannte kosmetische Inhaltsstoffe.

4. Die Zusammensetzung nach einem der Ansprüche 1 bis 3, wobei diese Zusammensetzung die Polymere A und B im Massenverhältnis Polymer A zu Polymer B = 1:3 bis 1:4 enthält.

5. Ein Kit of Parts umfassend
ein erstes Behältnis enthaltend Polymer A wie definiert in einem der vorhergehenden Ansprüche in einem Öl,
und ein zweites Behältnis enthaltend Polymer B wie definiert in einem der vorhergehenden Ansprüche in demselben Öl,
wobei die beiden Behältnisse optional auch zu einem Behältnis mit zwei Kammern verbunden sein können.

6. Ein Verfahren zur Herstellung eines Polymers A oder eines Polymers B, beide wie definiert in einem der vorhergehenden Ansprüche, umfassend
die Polymerisation der Monomere, welche das Polymer aufbauen, in einem Lösungsmittel, wobei dieses Lösungsmittel eine Mischung ist aus einem Öl und aus mindestens 4 Gew.-%, bezogen auf das Lösungsmittel, eines mono- oder difunktionellen Alkohols mit mindestens 4 C-Atomen.

7. Das Verfahren nach Anspruch 6, wobei der Alkohol ausgewählt wird aus der Gruppe bestehend aus Glycerinmonooleat, 2-Octyldodecan-1-ol und 2-Hexyldecan-1-ol.
